# EUROPÄISCHE PATENTSCHRIFT

(11) **EP 3 755 274 B1**
(45) Veröffentlichungstag und Bekanntmachung des Hinweises auf die Patenterteilung: **13.12.2023**
(21) Anmeldenummer: 19706962.8
(22) Anmeldetag: 20.02.2019
(51) Int. Cl.: A61F 2/50, A61F 2/60, A61F 2/68, A61F 5/01, F16F 9/32, F16F 9/516, A61F 2/64, A61F 2/74

(54) **HYDRAULIKDAEMPFER MIT VENTILBLOCK**
HYDRAULIC DAMPER WITH VALVE BLOCK
AMORTISSEUR HYDRAULIQUE ÉQUIPÉ D'UN BLOC DE VANNES

(30) Priorität: 21.02.2018 DE 102018103884
(43) Veröffentlichungstag der Anmeldung: 30.12.2020
(73) Patentinhaber: Otto Bock Healthcare Products GmbH, 1110 Wien (AT)
(72) Erfinder: MEJIA NINO, Juan Pablo, 1160 Wien (AT); GRAFL, Florian, 1040 Wien (AT)
(74) Vertreter: Gramm, Lins & Partner Patent- und Rechtsanwälte PartGmbB
(86) Internationale Anmeldenummer: PCT/EP2019/054223
(87) Internationale Veröffentlichungsnummer: WO 2019/162331

(56) Entgegenhaltungen:
- EP-A1- 3 015 736
- EP-A1- 3 208 488
- EP-A2- 1 215 413
- DE-A1- 10 344 152
- DE-A1-102015 201 528
- DE-U1- 20 313 999
- FR-E- 24 035

## Beschreibung

Die Erfindung betrifft einen Hydraulikaktuator mit einem Gehäuse, einer in dem Gehäuse angeordneten oder ausgebildeten Kammer, in der ein Kolben beweglich gelagert ist und die Kammer in eine Flexionskammer und eine Extensionskammer unterteilt, von der Flexionskammer und der Extensionskammer führt jeweils ein Kanal für Hydraulikfluid in das Gehäuse, um eine strömungstechnische Verbindung zwischen der Flexionskammer und der Extensionskammer auszubilden, wobei in der strömungstechnischen Verbindung zumindest ein Ventil angeordnet ist.

Hydraulikaktuatoren werden insbesondere bei orthopädietechnischen Einrichtungen wie Orthesen, Prothesen oder Rollstühlen eingesetzt. Sie können entweder als passive Aktuatoren ausgestaltet sein und als reine Dämpfer oder Sperren wirken, alternativ sind aktive Aktuatoren bekannt, die mit einem Energiespeicher oder Antrieb gekoppelt sind, um eine Verlagerung eines Gehäuses relativ zu einer Kolbenstange oder zu einem Abtriebselement zu ermöglichen. Hydraulikaktuatoren können als Linearaktuatoren oder Rotationsaktuatoren ausgebildet sein. Hydraulikaktuatoren weisen eine Hydraulikkammer auf, die mit einem Hydraulikfluid, insbesondere einem Öl, befüllt ist. Die Kammer innerhalb eines Gehäuses wird von einem Kolben in eine Extensionskammer und eine Flexionskammer unterteilt, die in ihrem Volumen veränderlich sind. Wird der Kolben in die eine oder andere Richtung bewegt, bei einer Linearhydraulik verschoben, bei einer Rotationshydraulik verschwenkt, wird in einer der Kammern das Druckniveau vergrößert, in der gegenüberliegenden Kammer das Druckniveau verringert. Um eine Bewegung überhaupt zu ermöglichen, ist eine strömungstechnische Verbindung zwischen der Flexionskammer und der Extensionskammer vorhanden. In der strömungstechnischen Verbindung, die als Hydraulikkanäle in dem Gehäuse oder über externe Leitungen ausgebildet sein kann, findet ein Volumenaustausch des Hydraulikfluides zwischen den Kammern statt. In den strömungstechnischen Verbindungen kann ein Drosselventil oder ein Stellventil angeordnet sein, über das der Strömungswiderstand von der Extensionskammer in die Flexionskammer und zurück veränderbar ist. Über die Einstellung des Strömungswiderstandes wird ein Bewegungswiderstand bereitgestellt, sodass beispielsweise bei einer Ausgestaltung des Hydraulikaktuators als Dämpfer in einem Prothesengelenk oder Orthesengelenk der Flexionswiderstand und/oder Extensionswiderstand veränderbar ist.

Problematisch ist der komplexe Aufbau des Hydraulikdämpfers mit einer Vielzahl von Kanälen, die in dem Gehäuse angeordnet sind und in denen Ventile eingesetzt werden müssen.

Die DE 203 13 999 U1 beschreibt ein künstliches Kniegelenk mit einem Hydraulikaktuator, der einen Kolben aufweist, der an einer Kolbenstange angeordnet ist und das Innere des Zylinders in eine erste Kammer und eine zweite Kammer teilt. Beide Kammern sind mit jeweils einem Kanal verbunden, der aus einem Gehäuse heraus und in einen Block hineinführt, in dem Ventile angeordnet sind.

Die FR 24 035 E beschreibt einen Zylinder mit einer Kolbenstange und einem Kolben, durch den der Zylinder in eine obere Kammer und eine untere Kammer unterteilt sind. Beide Kammern verfügen über einen Kanal, der in einen Ventilblock führt. Durch diesen Ventilblock und das darin enthaltene Ventil kann das Fluid aus der einen Kammer in die andere gelangen.

Die DE 10 2015 201 528 A1 betrifft eine Zylindervorrichtung, die als hydraulischer Stoßdämpfer zu verwenden ist und einen Zylinder mit einer Kolbenstange und einem Kolben aufweist, durch den der Innenraum des Zylinders in eine obere Kammer und in eine untere Kammer geteilt wird. Die Kanäle, die für eine Fluid-Kommunikation zwischen den beiden Kammern sorgen, verlaufen durch den Kolben.

Die EP 1 215 413 A2 betrifft einen regelbaren Schwingungsdämpfer für Kraftfahrzeuge mit einem Dämpfungselement für Zug- und/oder Druckdämpfung mit einer Ventileinrichtung als Regelventil, über die ein veränderbarer Dämpfungswiderstand eingestellt werden kann.

Die EP 3 208 488 A1 beschreibt einen Hydraulikaktuator mit einem Zylinder und einem an einer Kolbenstange befestigten, darin angeordneten Kolben und einem Leitungsblock, in dem zumindest ein Ventil angeordnet ist.

Die DE 103 44 152 A1 betrifft eine Kombinationsschraube zur Befestigung eines Ventilblockes an einer Hydraulikkolbenmaschine und zur Fixierung und/oder Verstellung einer verdrehbaren Steuereinrichtung, wobei die Kombinationsschraube zumindest in einem Teilbereich als Hohlschraube ausgebildet ist und der Hohlraum als Strömungskanal für Hydraulikflüssigkeit dient. Der Strömungskanal mündet in eine obere Austrittsöffnung. Zumindest ein seitlicher Auslasskanal führt von dem Strömungskanal an den äußeren Umfang der Schraube und stellt eine strömungstechnische Verbindung mit einem Ringkanal her, um eine Kurzschlussleitung in dem Ventilblock von einer Hochdruckseite zu einer Niederdruckseite zu ermöglichen.

Die EP 3 015 736 A1 betrifft einen Stoßdämpfer mit einem verschieblich in einem Zylinder an einer Kolbenstange gelagerten Kolben, wobei die Kolbenstange aus dem Zylindergehäuse herausragt. Eine Vorrichtung zur Erzeugung einer Dämpfungskraft steuert den Fluidstrom des Hydraulikfluides bei einer Verlagerung des Kolbens in dem Zylinder. Ein Hauptventil öffnet und schließt sich zur Steuerung des Fluidstromes, wobei durch das Hauptventil eine erste und eine zweite Drucckammer voneinander getrennt werden. Der Einlass in die erste Druckkammer wird über zwei Einlass-Rückschlagventile gesteuert. Der Auslass aus der zweiten Druckkammer wird durch zwei Auslass-Rückschlagventile gesteuert. Die Rückschlagventile sind in einem Gehäuse an der Oberseite des Zylindergehäuses angeordnet; das Gehäuse ist separat gefertigt und an dem Zylindergehäuse festgelegt.

Aufgabe der vorliegenden Erfindung ist es, eine Lösung für einen vereinfacht herzustellenden, stabilen Hydraulikaktuator bereitzustellen.

Erfindungsgemäß wird diese Aufgabe durch einen Hydraulikaktuator mit den Merkmalen des Hauptanspruches gelöst. Vorteilhafte Ausgestaltungen und Weiterbildungen der Erfindung sind in den Unteransprüchen, der Beschreibung sowie den Figuren aufgeführt.

Der erfindungsgemäße Hydraulikaktuator mit einem Gehäuse, einer in dem Gehäuse angeordneten oder ausgebildeten Kammer, in der ein Kolben beweglich gelagert ist und die Kammer in eine Flexionskammer und eine Extensionskammer unterteilt, wobei von der Flexionskammer und der Extensionskammer jeweils ein Kanal für Hydraulikfluid in das Gehäuse führt, um eine strömungstechnische Verbindung zwischen der Flexionskammer und der Extensionskammer auszubilden, und in der strömungstechnischen Verbindung zumindest ein Ventil angeordnet ist, sieht vor, dass die Kanäle aus dem Gehäuse heraus zu einem Ventilblock führen, in dem das zumindest eine Ventil angeordnet und strömungstechnisch mit den Kanälen gekoppelt ist, wobei der Ventilblock mit dem Gehäuse verbunden ist. Die Erfindung sieht vor, dass in dem Ventilblock Strömungskanäle angeordnet sind die zusammen mit den Kanälen eine strömungstechnische Verbindung zwischen der Flexionskammer und der Extensionkammer ausbilden, wobei alle Strömungskanäle in dem Ventilblock strömungstechnisch mit einer Bohrung gekoppelt sind. Über die Bohrung wird die strömungstechnische Verbindung aller Strömungskanäle in einer einfachen und zuverlässigen Art und Weise sichergestellt..

Die Erfindung sieht vor, dass in dem Ventilblock ein Ausgleichsvolumen angeordnet ist, das über einen der Strömungskanäle mit der Bohrung strömungstechnisch gekoppelt ist, was insbesondere bei einer Ausgestaltung des Hydraulikaktuators als Linearhydraulik mit einem Kolben und einer Kolbenstange vorteilhaft ist. Durch die Kolbenstange ändert sich je nach Position des Kolbens innerhalb der Kammer das Volumen, das mit dem Hydraulikfluid ausfüllbar ist. Um bei einem Einfahren des Kolbens und der Kolbenstange in die Kammer das überschüssige Hydraulikfluid innerhalb des Hydrauliksystems halten zu können, ist in dem Ventilblock das Ausgleichsvolumen angeordnet, um das Gehäusevolumen zu minimieren und innerhalb des Ventilblockes nach Möglichkeit sämtliche Mechaniken, Verstelleinrichtungen sowie gegebenenfalls Druckeinrichtungen zur Bereitstellung eines Druckes auf das Hydraulikfluid innerhalb des Ausgleichsvolumens bereitstellen zu können, damit bei einem Herausfahren der Kolbenstange aus der Kammer das Hydraulikfluid wieder hineingedrückt wird. In einer Ausführungsform der Erfindung ist es vorgesehen, dass ein weiteres Ausgleichsvolumen in dem Gehäuse angeordnet ist, beispielsweise als Teil einer Kammer.

Die Erfindung sieht in dem Ventilblock angeordnete Strömungskanäle vor, die alle miteinander strömungstechnisch gekoppelt sind. Durch die strömungstechnische Kopplung aller Strömungskanäle wird ein Mittelpunkt oder Strömungsmittelpunkt geschaffen, an dem sich alle das Fluid leitenden Kanäle treffen. Durch den Treffpunkt der Strömungskanäle kann die Anzahl der Strömungskanäle verringert werden, da alle Ventile miteinander koppelbar sind. Durch eine geeignete Anordnung von Stellventilen und Rückschlagventilen können zudem alle notwendigen Widerstandsmodifikationen einfach durchgeführt werden.

Die in dem Gehäuse ausgebildeten oder angeordneten Kanäle, die in die Extensionskammer beziehungsweise die Flexionskammer münden, bilden einen Teil der strömungstechnischen Verbindung aus, um bei einer Bewegung des Kolbens innerhalb der Kammer Hydraulikfluid von der Flexionskammer in die Extensionskammer und umgekehrt leiten zu können. Die Kanäle führen aus dem Gehäuse heraus in einen Ventilblock, in dem ebenfalls Kanäle angeordnet oder ausgebildet sind, wobei in diesen in dem Ventilblock angeordneten und ausgebildeten Strömungskanälen das zumindest eine Ventil angeordnet ist. Durch die Anordnung des zumindest einen Ventils in dem Ventilblock, der mit dem Gehäuse verbunden ist, vereinfacht sich die Herstellung des Hydraulikaktuators, da weniger Kanäle in der Gehäusewandung eingearbeitet werden müssen und insbesondere kleinere Kanäle eingearbeitet werden können, da innerhalb dieser Kanäle keine Ventile untergebracht werden müssen, die einen größeren Raum als die Kanäle einnehmen. Dadurch vergleichmäßigt sich die Lastverteilung innerhalb des Gehäuses, insbesondere der Gehäusewand, die Spannungen in dem Gehäuse werden verringert und es können schmalere Gehäusewände und insgesamt schmalere Gehäuse hergestellt werden, die leichter in der jeweiligen Einrichtung, beispielsweise einer orthopädietechnischen Einrichtung in Gestalt einer Orthese, Prothese oder eines Rollstuhls, untergebracht werden können. Der Hydraulikaktuator wird durch die Konstruktion mechanisch robuster, mit einer erhöhten Festigkeit gegenüber den herkömmlichen Konstruktionen.

Durch die Fertigung des Ventilblockes als separates Bauteil und die Anordnung an dem Gehäuse kann eine modulare Fertigung erreicht werden. Darüber hinaus können Änderungen an oder in dem Ventilblock leichter umgesetzt werden. Modifikationen lassen sich leichter verwirklichen und ein Baukastensystem kann etabliert werden, bei dem mehrere Gehäusegrundkörper mit mehreren Ventilblöcken kombiniert werden können, um unterschiedliche Baureihen zusammenstellen zu können.

Vorteilhafterweise ist der Ventilblock lösbar an dem Gehäuse festgelegt, um die Variation und die Montage zu erleichtern. Darüber hinaus werden durch die lösbare Festlegung des Ventilblockes an dem Gehäuse eine Wartung erleichtert und Reparaturmöglichkeiten verbessert.

In einer Weiterbildung der Erfindung ist vorgesehen, dass in dem Ventilblock zumindest zwei Stellventile und zumindest zwei Rückschlagventile angeordnet sind, um im Rahmen einer Sternarchitektur der Kanäle innerhalb des Ventilblockes die Strömungsrichtungen beliebig zu beeinflussen und darüber hinaus sowohl bei der Extension als auch bei der Flexion einstellbare Strömungswiderstände bereitstellen zu können.

Erfindungsgemäß ist in der Bohrung eine Schraube mit Verbindungskanälen eingesetzt, die die Strömungskanäle miteinander verbinden. Die Verbindungskanäle innerhalb der Schraube verbinden bevorzugt die Strömungskanäle, die zu den Kanälen der Flexionskammer und Extensionskammer sowie zu dem Ausgleichsvolumen führen. Über die Schraube ist der Ventilblock an dem Gehäuse festgelegt, sodass die Schraube nicht nur die mechanische Verbindung von dem Ventilblock mit dem Gehäuse ausbildet, sondern auch die hydraulische Verbindung zwischen der Extensionskammer und der Flexionskammer sowie den Ventilen und dem Ausgleichsvolumen herstellt. Die Schraube kann der Sternpunkt oder der Sammelpunkt aller Strömungskanäle sein oder einen solchen Sammelpunkt ausbilden, an dem alle innerhalb des Ventilblockes angeordneten oder ausgebildeten Kanäle strömungstechnisch miteinander gekoppelt sind. Die Kanäle können sich auf dem Weg von einem Einlass in den Ventilblock zu einem Auslass aus dem Ventilblock teilen oder zusammengeführt werden, ebenso kann in einem oder in mehreren Kanälen ein Sperrventil angeordnet sein, das einen Durchlass in einer Strömungsrichtung sperrt, in der anderen Strömungsrichtung jedoch zulässt. Alternativ kann die Schraube über einen Strömungskanal, beispielsweise einen Niederdruckkanal, mit diesem Sammelpunkt oder Sternpunkt verbunden sein. Durch die Ausgestaltung der Schraube als mechanische und hydraulische Verbindung zwischen dem Gehäuse und dem Ventilblock kann die Baugröße sowohl des Ventilblockes als auch des Gehäuses des Hydraulikaktuators verkleinert werden, da die Komplexität des Gehäuses und auch des Ventilblockes reduziert wird.

In dem Ventilblock kann jeweils ein Anschluss für einen Kanal aus der Flexionskammer und der Extensionskammer ausgebildet sein, wobei dieser Anschluss als Kanal oder Bohrung ausgebildet sein kann, die korrespondierend zu dem jeweiligen Kanal aus der Flexionskammer oder Extensionskammer angeordnet ist, wenn der Ventilblock an dem Gehäuse montiert ist. Der Anschluss kann sich in zwei Strömungskanäle aufteilen, von denen einer mit einem verstellbaren Drosselventil und der andere mit einem Rückschlagventil versehen ist. Innerhalb des Ventilblockes sind somit zumindest vier Strömungskanäle angeordnet, von denen sich zwei jeweils zu einem Anschluss zusammenfinden und zu dem Kanal führen, der zu der Extensionskammer oder Flexionskammer führt. Von dem jeweiligen Anschluss zweigen somit paarweise Strömungskanäle ab, in denen Servoventile und Rückschlagventile angeordnet sind und die zu dem gemeinsamen Strömungspunkt oder Sternpunkt führen, in dem die Schraube mit denen entsprechenden Verbindungskanälen angeordnet sein kann.

Der Kopplungsstelle der Strömungskanäle oder dem Sternpunkt, an dem sich die Strömungskanäle treffen, ist in Zuströmrichtung ein Drosselventil vorgelagert, sodass die Kopplungsstelle oder der Sternpunkt nur mit Hydraulikfluid beaufschlagt wird, das bereits durch ein Drosselventil hindurch gegangen ist. Dadurch wird gewährleistet, dass bei einem nicht voll geöffneten Drosselventil nur ein reduzierter Druck an der Kopplungsstelle anliegt und von der Hochdruckseite zur Niederdruckseite strömen kann.

Um eine minimale mechanische Störung in dem Gehäuse auszubilden, ist in einer Variante der Erfindung vorgesehen, dass aus dem Gehäuse genau zwei Kanäle herausführen, einer von der Extensionskammer und einer von der Flexionskammer. Grundsätzlich können die Kanäle an einer beliebigen Stelle des Gehäuses herausführen, wo sich der Ventilblock leicht befestigen kann, bevorzugt wird der Ventilblock an der Stirnseite des Gehäuses angeordnet, da hier eine besonders einfache Montage aufgrund der glattflächigen Anlage des Ventilblockes an der Stirnseite erreicht werden kann, wenn der Kolben und der Zylinder rund ausgebildet sind.

Der Hydraulikaktuator ist bevorzugt als ein Linearhydraulikaktuator mit einer zylindrischen Kammer und einem längsverschieblich darin gelagerten Kolben und einer aus dem Gehäuse herausgeführten Kolbenstange ausgebildet.

Die Erfindung betrifft ebenfalls einen Ventilblock zur Befestigung an einem Hydraulikaktuator, wobei in dem Ventilblock zumindest ein Ventil angeordnet und strömungstechnisch mit Kanälen aus einem Gehäuse koppelbar und der Ventilblock mit dem Gehäuse verbindbar ist.

In einer Weiterbildung der Erfindung ist der Ventilblock wie oben beschrieben ausgebildet.

Weiterhin betrifft die Erfindung eine Schraube mit Verbindungskanälen, die Strömungskanäle innerhalb des Ventilblocks miteinander verbinden.

Nachfolgend wird ein Ausführungsbeispiel der Erfindung anhand der beigefügten Figuren näher erläutert.

Es zeigen:
- Figur 1 -: eine schematische Darstellung eines Hydraulikaktuators in Gestalt eines Dämpfers mit einem montierten Ventilblock;
- Figur 2 -: eine schematische Darstellung einer Variante der Erfindung bei einer Flexionsbewegung;
- Figur 3 -: eine Ausgestaltung der Erfindung bei einer Extensionsbewegung;
- Figur 4 -: eine perspektivische Darstellung einer Verbindungsschraube;
- Figur 5 -: eine Schnittdarstellung der Verbindungsschraube gemäß Figur 4,
- Figur 6 -: ein Anwendungsbeispiel in einer Orthese; sowie
- Figur 7 -: ein Anwendungsbeispiel in einer Prothese.

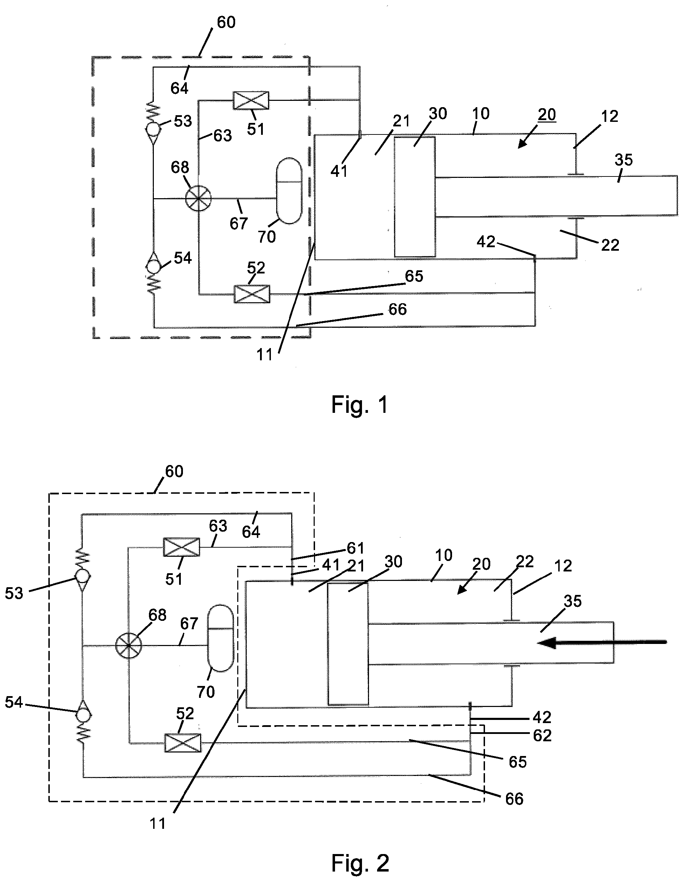

In der Figur 1 ist in einer schematischen Darstellung ein Hydraulikaktuator 100 mit einem Gehäuse 10 dargestellt, in dem eine zylindrische Kammer 20 ausgebildet ist, in der ein Kolben 30 längsbeweglich gelagert ist. Der Kolben 30 ist entlang einer Längserstreckung einer Kolbenstange 35 innerhalb der Kammer 20 verschieblich gelagert und unterteilt die Kammer 20 in eine Flexionskammer 21 und eine Extensionskammer 22. Der Kolben 30 verringert das Volumen der Flexionskammer 21, wenn der Kolben 30 mit der Kolbenstange 35 in die Kammer 20 eingefahren wird. Wird die Kolbenstange 35 mit dem Kolben 30 aus der Kammer 20 herausgefahren, verringert sich das Volumen der Extensionskammer 22. Extension oder Flexion treten auf, wenn der Hydraulikaktuator 100 an gelenkig miteinander verbundenen Komponenten befestigt ist. Ist beispielsweise die Kolbenstange 35 an einem Oberteil eines orthopädischen oder prothetischen Gelenkes und das Gehäuse 10 an einem Unterteil, beispielsweise einem Oberschenkelteil und einem Unterschenkelteil, angeordnet, die dann durch ein Prothesenkniegelenk oder Orthesenkniegelenk miteinander verbunden sind, findet bei einer einfahrenden Bewegung der Kolbenstange 35 eine Flexion und bei einer ausfahrenden Bewegung eine Extension statt. Der Kolben 30 ist gegenüber der Innenwand der Kammer 20 abgedichtet, sodass Hydraulikfluid, das in der Kammer 20 vorhanden ist, bei einer Bewegung des Kolbens 30 innerhalb der Kammer 20 von der Flexionskammer 21 in die Extensionskammer 22 bei einer Flexionsbewegung und von der Extensionskammer 22 in die Flexionskammer 21 bei einer Extensionsbewegung strömen muss. Sowohl an der Flexionskammer 21 als auch an der Extensionskammer 22 ist ein Kanal 41, 42 für Hydraulikfluid vorgesehen, durch den Hydraulikfluid aus der jeweiligen Kammer 21, 22 einströmen und ausströmen kann. Die Strömungsrichtung ist von der Bewegung des Kolbens 30 abhängig. In dem dargestellten Ausführungsbeispiel führt genau ein Kanal 41, 42 aus der Flexionskammer 21 beziehungsweise Extensionskammer 22 in das Gehäuse 10. An dem Gehäuse 10 ist ein Ventilblock 60 befestigt, der als separate Komponente ausgebildet ist. Der Ventilblock 60 ist schematisch durch die gestrichelte Umrandung dargestellt. Innerhalb des Ventilblocks 60 sind Strömungskanäle 63, 64, 65, 66, 67 ausgebildet, die zusammen mit den Kanälen 41, 42 eine strömungstechnische Verbindung zwischen der Flexionskammer 21 und der Extensionskammer 22 ausbilden. In dem Ausführungsbeispiel gemäß Figur 1 sind an der Schnittstelle zwischen dem Ventilblock 60 und dem Gehäuse 10 zwei Strömungskanalpaare 63, 64, 65, 66 ausgebildet, die korrespondierend zu einem in zwei Abschnitte aufgeteilten Kanal 41, 42 positioniert sind. Die Kanäle 41, 42 von der Flexionskammer 21 beziehungsweise Extensionskammer 22 sind in dem Ausführungsbeispiel gemäß Figur 1 innerhalb des Gehäuses 10 auf zwei Kanalabschnitte aufgeteilt, sodass diese korrespondierend zu dem Strömungskanälen 63, 64 beziehungsweise 65, 66 innerhalb des Ventilblockes 60 positioniert sind, wenn dieser an dem Gehäuse 10 festgelegt ist. In der dargestellten Ausführungsform des Gehäuses 10 führt nur genau ein Kanal 41, 42 aus der Flexionskammer 21 oder Extensionskammer 22 aus dem Gehäuse 10 heraus, sodass das Gehäuse 10 selbst nur mit einem minimalen Materialverlust für die Ausbildung des jeweiligen Kanals 41, 42 belastet ist. Dadurch verringern sich die Spannungen innerhalb des Gehäuses 10, da nur eine geringe Anzahl an Kanälen 41, 42, nämlich zwei, die zudem sehr kurz ausgebildet sein können, ausgebildet werden muss.

Innerhalb des Ventilblockes 60 führen die Strömungskanäle 63, 64, 65, 66 zu einer Bohrung 68 oder einem Sternpunkt, an dem alle Strömungskanäle 63, 64, 65, 66, innerhalb des Ventilblockes 60 zusammenlaufen und eine strömungstechnische Verbindung untereinander herstellen. Innerhalb des Ventilblockes 60 ist auch noch ein Ausgleichsvolumen 70 ausgebildet, das zum Ausgleich von Volumenschwankungen notwendig ist. Das Ausgleichsvolumen 70 ist über einen Strömungskanal 67 mit der Bohrung 68 oder dem Sternpunkt der Strömungskanäle 63, 64, 65, 66, 67 strömungstechnisch gekoppelt.

Von der Schnittstelle des Ventilblockes 60 bis zu der Bohrung 68 oder dem strömungstechnischen Sternpunkt sind in den getrennten Strömungskanälen 63, 64, 65, 66 Stellventile 51, 52 und Rückschlagventile 53, 54 angeordnet. Von der Flexionskammer 21 führt ein erster Strömungskanal 63 über ein erstes Stellventil 51 zu der gemeinsamen Bohrung 68 ebenso wie ein zweiter Strömungskanal 64, der mit einem ersten federbelasteten Rückschlagventil 53 ausgestattet ist. Das erste Rückschlagventil 53 sperrt einen Fluiddurchgang von der Flexionskammer 21 in Richtung auf die gemeinsame Bohrung 68 und auch zu der Extensionskammer 22. Bei einer Flexion oder bei einem Einfahren des Kolbens 30 in die Kammer 20 zur Verringerung des Volumens innerhalb der Flexionskammer 21 muss somit das gesamte Hydraulikfluid durch das erste Stellventil 51 hindurchtreten, bevor es über die gemeinsame Bohrung 68 oder den Sternpunkt an das Ausgleichsvolumen 70 oder aber zu den spiegelbildlich angeordneten Stellventilen 52, 54 geleitet wird. Das zweite Stellventil 52 und das zweite Rückschlagventil 54 sind über Strömungskanäle 65, 66 mit dem Kanal 42 verbunden, der zur Extensionskammer 22 führt. Von der gemeinsamen Bohrung 68 oder dem strömungstechnischen Sternpunkt 68 führt ein Strömungskanal über das zweite Stellventil 52 zu der Extensionskammer 22 und ein weiterer Strömungskanal 66, in dem das zweite federbelastete Rückschlagventil 54 angeordnet ist, zu der Extensionskammer 22 oder den Kanälen innerhalb des Gehäuses 10, die zu dem Kanal 42 zur Extensionskammer 22 führen. Die beiden Rückschlagventile 53, 54 sind über einen Verbindungskanal miteinander und mit der gemeinsamen Bohrung 68 gekoppelt. Alternativ werden die den Rückschlagventilen 53, 54 zugeordneten Strömungskanäle 64, 66 innerhalb des Ventilblockes 60 getrennt zu der gemeinsamen Bohrung 68 oder dem strömungstechnischen Sternpunkt geleitet. Die Rückschlagventile 53, 54 sperren eine Hochdruckleitung zu dem gemeinsamen hydraulischen Sternpunkt 68 und erlauben eine Strömung in eine Niederdruckkammer von dem Sternpunkt 68 erst, nachdem ein hochdruckseitiges Stellventil 51, 52 passiert worden ist.

Die gemeinsame Bohrung 68 muss keine zylindrische Bohrung sein, wesentlich ist, dass ein Punkt oder ein strömungstechnischer Raum gebildet wird, an dem sich alle Niederdruckleitungen treffen.

Als Hochdruckleitung gelten die Leitungen oder Strömungskanäle, in die direkt Hydraulikfluid aus der jeweiligen Kammer hineingedrückt wird, als Niederdruckleitung gelten diejenigen Kanäle, die in Strömungsrichtung gesehen hinter einem Stellventil 51, 52 oder einem Rückschlagventil 53, 54 liegen und entweder zu dem Sternpunkt 68, dem Ausgleichsvolumen 70 oder zu der jeweiligen Niederdrucckammer führen, in die das Hydraulikfluid einströmt.

Figur 2 zeigt eine Variante der Erfindung, bei der der Ventilblock 60 nur über zwei Kanalschnittstellen mit dem Gehäuse 10 gekoppelt ist. Ein erster Kanal 41 wird in einem montierten Zustand einem ersten Anschluss 61 und ein zweiter Kanal 42 wird einem zweiten Anschluss 62 gegenüberliegend positioniert und so angeordnet, dass Hydraulikfluid von der Flexionskammer 21 in die Extensionskammer 22 strömen kann. In dem dargestellten Ausführungsbeispiel erstrecken sich die Kanäle 41, 42 radial nach außen aus dem Gehäuse 10. Alternativ ist es möglich und vorgesehen, dass die Kanäle 41, 42 an einer Stirnseite 11, 12 des Gehäuses 10 herausgeführt sind und zumindest teilweise parallel zu der Bewegungsrichtung des Kolbens 30 verlaufen. Der Ventilblock 60 wird dann stirnseitig an das Gehäuse 10 angeschraubt oder daran befestigt. Wird alternativ der Ventilblock 60 seitlich an dem Gehäuse 10 befestigt, verringert dies die Baulänge des Hydraulikaktuator, so dass eine solche Variante gewählt wird, wenn wenig Bauraum zur Verfügung steht. Innerhalb des Ventilblocks 60 teilt sich der jeweilige Anschluss 61, 62 in zwei Strömungskanäle 63, 64, 65, 66 auf. Der Kolben 30 wird über die Kolbenstange 35 in Pfeilrichtung nach links bewegt, sodass die Flexionskammer 21 verkleinert und das in der Flexionskammer 21 befindliche Hydraulikfluid unter Druck gesetzt wird. Das Hydraulikfluid strömt aus der Flexionskammer 21 durch den Kanal 41 in den Anschluss 61 und von dort in die beiden Strömungskanäle 63 ,64. Aufgrund des ersten Sperrventils 53 wird kein Hydraulikfluid durch den oberen Strömungskanal 64 hindurchtreten. Das Hydraulikfluid wird daher durch das erste Stellventil 51 in Richtung auf die Bohrung 68 oder den gemeinsamen Strömungssternpunkt geleitet. Von der Bohrung 68 tritt das nach dem Durchtritt durch das erste Stellventil 51 gedrosselte Hydraulikfluid je nach Ventilstellung entweder durch das Stellventil 52, durch das Rückschlagventil 54 oder durch das zweite Stellventil 52 als auch durch das zweite Rückschlagventil 54. Nach dem Durchgang durch das jeweilige Ventil 52, 54 wird das Hydraulikfluid durch die Kanäle 65, 66 zu dem Anschluss 62 und von dort durch den Kanal 42 in die Extensionskammer 22 geleitet. Da bei der Flexionsbewegung, also bei der Verlagerung des Kolbens 30 nach links, die Kolbenstange 35 in die Extensionskammer 22 eintritt, verringert sich das zur Verfügung stehende Kammervolumen um das Volumen der eindringenden Kolbenstange 35, sodass ein Teil des Hydraulikfluid aus der Flexionskammer 21 in das Ausgleichsvolumen 70 durch den Strömungskanal 67 strömt. Über das erste Stellventil 51 ebenso wie über die Federbelastung des zweiten Rückschlagventils 54 als auch das zweite Stellventil 52 kann der Dämpfungswiderstand in Flexionsrichtung eingestellt werden.

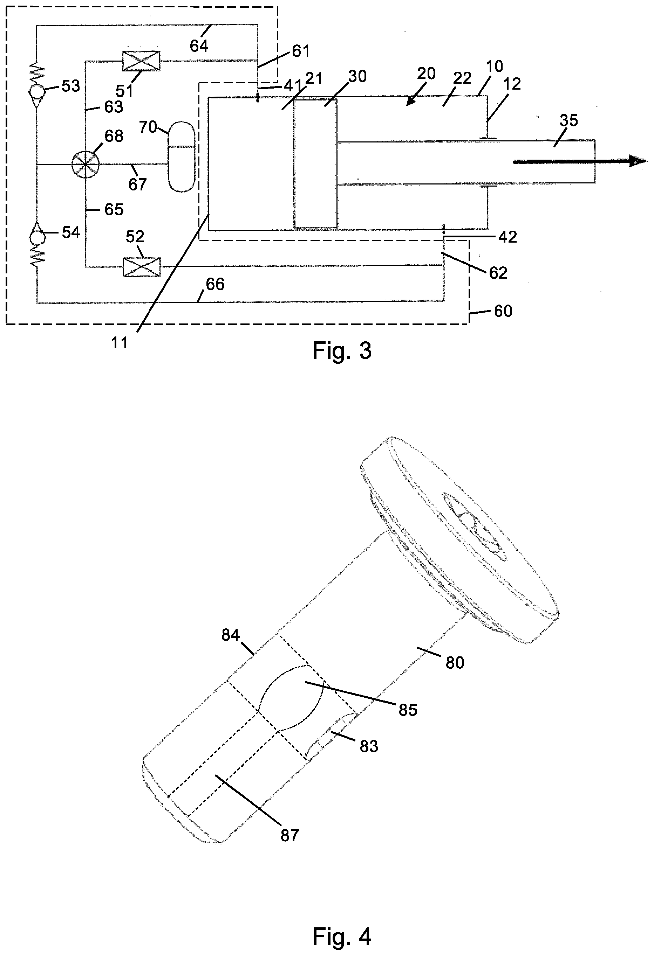

Bei einer umgekehrten Bewegung, die in der Figur 3 dargestellt ist, wird die Kolbenstange 35 aus der Extensionskammer 22 herausgezogen, der Kolben 30 wird nach rechts bewegt. Somit wird eine Kraft auf das Hydraulikfluid innerhalb der Extensionskammer 22 ausgeübt, und das unter Druck stehende Hydraulikfluid tritt durch den Kanal 42 in den Anschluss 62 von dort in die Strömungskanäle 65 und 66. Das zweite Rückschlagventil 54 sperrt den Durchgang durch den Strömungskanal 66, sodass das Hydraulikfluid aus der Extensionskammer 22 durch das zweite Stellventil 52 und den Strömungskanal 65 zur Bohrung 68 strömt. Von dort gelangt es durch das erste Rückschlagventil 53 und das erste Stellventil 51 durch die Kanäle 63, 64 zu dem ersten Anschluss 61 und in die Flexionskammer 21. Das fehlende Volumen an Hydraulikfluid wird aus dem druckbelasteten Ausgleichsvolumen 70 bereitgestellt.

Figur 4 zeigt eine mögliche Ausgestaltung der Bohrung 68 oder des strömungstechnischen Sternpunktes in Gestalt einer Schraube 80, in der vier Verbindungskanäle zur Verbindung der Strömungskanäle 63, 64, 65, 66, 67 ausgebildet sind. Die Verbindungskanäle 83, 84, 85 sind sichtbar, der vierte Verbindungskanal, der den Strömungskanal 66 von dem zweiten Rückschlagventil 54 mit den übrigen Strömungskanälen 63, 64, 65, 67 verbindet, ist nicht gezeigt. Ebenfalls ist es möglich, nur drei Verbindungskanäle 83, 84, 85 vorzusehen, wenn die Strömungskanäle 66, 64 nach den Rückschlagventilen 53, 54 zu einem gemeinsamen Kanal zusammengeführt werden. Es ergibt sich eine T-artige Kreuzung der Verbindungskanäle 83, 84, 85. Die Verbindungskanäle sind radial zu der Längserstreckung des Schraubenschaftes ausgebildet. In dem dargestellten Ausführungsbeispiel treffen sich die Verbindungskanäle 83, 84, 85 mittig innerhalb des Schraubenschaftes. Zusätzlich führt ein in Längserstreckung des Schraubenschaftes ausgebildeter Verbindungskanal 87 zu dem Ausgleichsvolumen 70, beziehungsweise zu dem Strömungskanal 67, der zu dem Ausgleichsvolumen 70 führt. Somit wird durch die Schraube 80 einerseits eine strömungstechnische Verbindung zwischen allen Strömungskanälen 63, 64, 65, 66, 67 und andererseits eine mechanische Verbindung zwischen dem Ventilblock 60 und dem Gehäuse 10 ausgebildet. Die Schraube 80 bildet die Verbindung mindestens zweiter Niederdruckkanäle und kann gleichzeitig eine mechanische Verbindung mehrerer Komponenten einer Linearhydraulik bereitstellen. Die Schraube 80 muss nicht unbedingt der strömungstechnische Sternpunkt oder Verbindungspunkt sein, sondern kann auch über einen Niedrigdruckkanal mit dem Sternpunkt oder der Bohrung 68 verbunden sein.

Durch eine Kombination einer mechanischen Festlegung des Ventilblocks 60 an oder in dem Gehäuse 10 und einer strömungstechnischen Verbindung aller Kanäle in der Schraube 80 wird die Baugröße des Hydraulikaktuators verkleinert. Darüber hinaus wird die Komplexität des Zylinders mit dem Gehäuse 10 und dem darin befindlichen Kolben 30 stark reduziert. Nur zwei Kanäle 41, 42 führen aus dem Gehäuse 10 heraus und leiten das Hydraulikfluid in den Ventilblock 60, in dem die Stellventile 51, 52, die Rückschlagventile 53, 54 und das Ausgleichsvolumen 70 angeordnet sind.

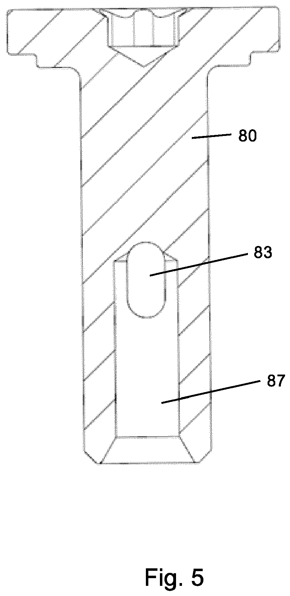

Figur 5 zeigt die Schraube 80 in einer Schnittdarstellung. Es ist die Ausgestaltung des Verbindungskanals 87 in Längserstreckung des Schraubenschaftes und die senkrechte Orientierung eines Verbindungskanals 83, der radial zu der Längserstreckung der Schraube 80 orientiert ist, dargestellt.

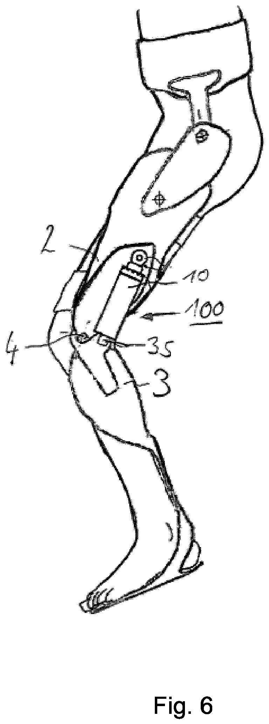

Figur 6 zeigt ein Anwendungsbeispiel einer Anbindung eines Hydraulikaktuators 100 an einer Orthese. In dem dargestellten Ausführungsbeispiel überbrückt die Orthese sämtliche Gelenke der unteren Extremität. Der Hydraulikaktuator 100 ist mit einem distalen Befestigungspunkt an einem Oberschenkelteil 2 befestigt, das über Befestigungsgurte an einem Oberschenkel festgelegt ist. Das Oberschenkelteil 2 ist proximal gelenkig mit einem Hüftgurt oder einer Hüftschale verbunden und distal über eine Schwenkachse 4 gelenkig mit einem Unterschenkelteil 3, das eine Fußabstützung aufweist. Das Unterteil 3 lässt sich um die Schwenkachse 4 in Extensionsrichtung und Flexionsrichtung relativ zu dem Oberschenkelteil 2 verschwenken. Der Hydraulikaktuator 100 ist sowohl an dem Oberschenkelteil 2 als auch an dem Unterschenkelteil 3 gelagert. An dem Unterschenkelteil 3 ist die Kolbenstange 35 befestigt, die aus dem Gehäuse 10 herausragt. Schwenkbewegungen zwischen dem Oberschenkelteil 2 und dem Unterschenkelteil 3 werden durch den Hydraulikaktuator 100 unterstützt oder gedämpft.

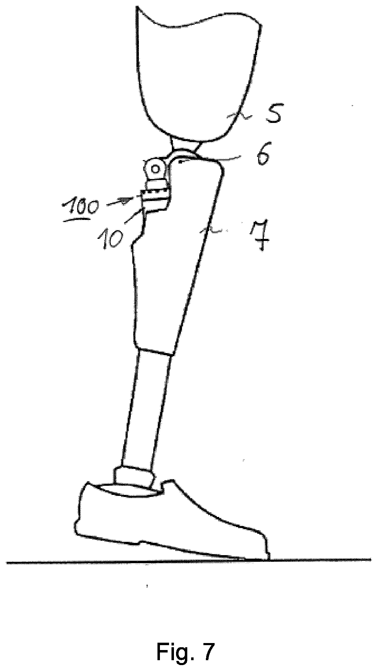

Ein alternatives Anwendungsbeispiel ist in der Figur 7 gezeigt, bei der der Hydraulikaktuator 100 zwischen einem Prothesenoberteil 5 in Gestalt eines Oberschenkelschaftes und einem Unterschenkelteil 7 angeordnet ist. Das Oberteil 5 und das Unterteil 7 sind in einem Prothesenkniegelenk um eine Schwenkachse 6 verschwenkbar miteinander verbunden. Das Gehäuse 10 mit einer Lageraufnahme ist an einem Ausleger an dem Oberteil 5 befestigt, während die nicht sichtbare Kolbenstange an dem Unterschenkelteil 7 gelagert ist. Bei einer Flexion und bei einer Extension wird der Hydraulikaktuator 100 mit Linearkräften beaufschlagt und die Kolbenstange in das Gehäuse 10 eingefahren oder herausgezogen.

### Bezugszeichenliste:

- 10: Gehäuse
- 11: Stirnseite
- 12: Stirnseite
- 20: Kammer
- 21: Flexionskammer
- 22: Extensionskammer
- 30: Kolben
- 35: Kolbenstange
- 41: Kanal
- 42: Kanal
- 51: erstes Stellventil
- 52: zweites Stellventil
- 53: erstes Rückschlagventil
- 54: zweites Rückschlagventil
- 60: Ventilblock
- 61: erster Anschluss
- 62: zweiter Anschluss
- 63: Strömungskanal
- 64: Strömungskanal
- 65: Strömungskanal
- 66: Strömungskanal
- 67: Strömungskanal
- 68: Bohrung
- 70: Ausgleichsvolumen
- 80: Schraube
- 83: Verbindungskanal
- 84: Verbindungskanal
- 85: Verbindungskanal
- 87: Verbindungskanal
- 100: Hydraulikaktuator

## Patentansprüche

1. Hydraulikaktuator mit einem Gehäuse (10), einer in dem Gehäuse (10) angeordneten oder ausgebildeten Kammer (20), in der ein Kolben (30) beweglich gelagert ist und die Kammer (20) in eine Flexionskammer (21) und eine Extensionskammer (22) unterteilt,
wobei von der Flexionskammer (21) und der Extensionskammer (22) jeweils ein Kanal (41, 42) für Hydraulikfluid in das Gehäuse (10) führt, um eine strömungstechnische Verbindung zwischen der Flexionskammer (21) und der Extensionskammer (22) auszubilden,
wobei in der strömungstechnischen Verbindung zumindest ein Ventil (51, 52, 53, 54) angeordnet ist,
wobei die Kanäle (41, 42) aus dem Gehäuse (10) heraus zu einem Ventilblock (60) führen, in dem das zumindest eine Ventil (51, 52, 53, 54) angeordnet und strömungstechnisch mit den Kanälen (41, 42) gekoppelt ist,
wobei der Ventilblock (60) mit dem Gehäuse (10) verbunden ist,
wobei in dem Ventilblock (60) Strömungskanäle (63, 64, 65, 66, 67) angeordnet sind, die zusammen mit den Kanälen (41, 42) eine strömungstechnische Verbindung zwischen der Flexionskammer (21) und der Extensionkammer (22) ausbilden,
wobei alle Strömungskanäle (63, 64, 65, 66, 67) in dem Ventilblock (60) strömungstechnisch mit einer Bohrung (68) gekoppelt sind, **wobei** ein Ausgleichsvolumen (70) in dem Ventilblock (60) angeordnet ist, das über einen der Strömungskanäle (67) mit der Bohrung (68) strömungstechnisch gekoppelt ist, wobei
in der Bohrung (68) eine Schraube (80) mit Verbindungskanälen (83, 84, 85, 87) eingesetzt ist, die die Strömungskanäle (63, 64, 65, 66, 67) miteinander verbinden, **dadurch gekennzeichnet, dass**
die Schraube (80) den Ventilblock (60) an dem Gehäuse (10) festlegt.

2. Hydraulikaktuator nach Anspruch 1, **dadurch gekennzeichnet, dass** der Ventilblock (60) an dem Gehäuse (10) lösbar festgelegt ist.

3. Hydraulikaktuator nach Anspruch 1 oder 2, **dadurch gekennzeichnet, dass** in dem Ventilblock (60) zumindest zwei Stellventile (51, 52) und zumindest zwei Rückschlagventile (53, 54) angeordnet sind.

4. Hydraulikaktuator nach einem der voranstehenden Ansprüche, **dadurch gekennzeichnet, dass** in dem Ventilblock (60) je ein Anschluss (61, 62) für einen Kanal (41, 42) aus der Flexionskammer (21) und der Extensionskammer (22) ausgebildet ist und sich der Anschluss (61, 62) in zwei Strömungskanäle (63, 64; 65, 66) aufteilt, von denen einer mit einem verstellbaren Drosselventil (51, 52) und der andere mit einem Rückschlagventil (53, 54) versehen ist.

5. Hydraulikaktuator nach einem der voranstehenden Ansprüche, **dadurch gekennzeichnet, dass** der Kopplungsstelle der Strömungskanäle (63, 64, 65, 66, 67) in Zuströmrichtung ein Drosselventil (51, 52) vorgelagert ist.

6. Hydraulikaktuator nach einem der voranstehenden Ansprüche, **dadurch gekennzeichnet, dass** aus dem Gehäuse (10) genau zwei Kanäle (41, 42) herausführen.

7. Hydraulikaktuator nach einem der voranstehenden Ansprüche, **dadurch gekennzeichnet, dass** der Ventilblock (60) an einer Stirnseite (11, 12) des Gehäuses (10) angeordnet ist.

8. Hydraulikaktuator nach einem der voranstehenden Ansprüche, **dadurch gekennzeichnet, dass** der Hydraulikaktuator als Linearhydraulikaktuator mit einer zylindrischen Kammer (20) und einem längsverschieblich darin gelagerten Kolben (30) und einer aus dem Gehäuse (10) herausgeführten Kolbenstange (35) ausgebildet ist.

## Claims

1. A hydraulic actuator having a housing (10), a chamber (20) which is arranged or formed in the housing (10) and in which a piston (30) is movably mounted and divides the chamber (20) into a flexion chamber (21) and an extension chamber (22),
wherein a channel (41, 42) for hydraulic fluid leads from each of the flexion chamber (21) and the extension chamber (22) into the housing (10) in order to produce a fluidic connection between the flexion chamber (21) and the extension chamber (22),
wherein at least one valve (51, 52, 53, 54) is arranged in the fluidic connection,
wherein the channels (41, 42) lead out of the housing (10) to a valve block (60) in which the at least one valve (51, 52, 53, 54) is arranged and fluidically coupled with the channels (41, 42),
wherein the valve block (60) is connected to the housing (10),
wherein flow channels (63, 64, 65, 66, 67) are arranged in the valve block (60), the flow channels (63, 64, 65, 66, 67) forming a fluidic connection between the flexion chamber (21) and the extension chamber (22) together with the channels (41, 42),
wherein all the flow channels (63, 64, 65, 66, 67) in the valve block (60) are fluidically coupled with a bore (68), wherein
a compensation volume (70) is arranged in the valve block (60), the compensation volume (70) being fluidically coupled with the bore (68) via one of the flow channels (67), wherein
a screw (80) having connecting channels (83, 84, 85, 87) which connect the flow channels (63, 64, 65, 66, 67) to one another is inserted in the bore (68), **characterized in that**
the screw (80) fixes the valve block (60) to the housing (10).

2. The hydraulic actuator as claimed in claim 1, **characterized in that** the valve block (60) is detachably fastened to the housing (10).

3. The hydraulic actuator as claimed in claim 1 or 2, **characterized in that** at least two control valves (51, 52) and at least two non-return valves (53, 54) are arranged in the valve block (60).

4. The hydraulic actuator as claimed in one of the preceding claims, **characterized in that** a connection (61, 62) for each channel (41, 42) from the flexion chamber (21) and the extension chamber (22) is formed in the valve block (60) and the connection (61, 62) is divided into two flow channels (63, 64; 65, 66), of which one is provided with an adjustable throttle valve (51, 52) and the other with a non-return valve (53, 54).

5. The hydraulic actuator as claimed in one of the preceding claims, **characterized in that** a throttle valve (51, 52) is located upstream, in the inflow direction, of the coupling point of the flow channels (63, 64, 65, 66, 67).

6. The hydraulic actuator as claimed in one of the preceding claims, **characterized in that** exactly two channels (41, 42) lead out of the housing (10).

7. The hydraulic actuator as claimed in one of the preceding claims, **characterized in that** the valve block (60) is arranged on an end face (11, 12) of the housing (10).

8. The hydraulic actuator as claimed in one of the preceding claims, **characterized in that** the hydraulic actuator is configured as a linear hydraulic actuator with a cylindrical chamber (20) and a piston (30) mounted therein in a longitudinally displaceable manner, and a piston rod (35) which is guided out of the housing (10).

## Revendications

1. Actionneur hydraulique comprenant un boîtier (10), une chambre (20) disposée ou réalisée dans le boîtier (10), dans laquelle un piston (30) est monté de façon mobile et subdivise la chambre (20) en une chambre de flexion (21) et en une chambre d'extension (22),
dans lequel
un canal (41, 42) respectif pour un fluide hydraulique mène de la chambre de flexion (21) et de la chambre d'extension (22) jusque dans le boîtier (10), afin d'établir une communication fluidique entre la chambre de flexion (21) et la chambre d'extension (22),
au moins une vanne (51, 52, 53, 54) est disposée dans la communication fluidique,
les canaux (41, 42) mènent hors du boîtier (10) vers un bloc de vanne (60) dans lequel ladite au moins une vanne (51, 52, 53, 54) est disposée et couplée fluidiquement aux canaux (41, 42),
le bloc de vanne (60) est relié au boîtier (10),
des canaux d'écoulement (63, 64, 65, 66, 67) sont disposés dans le bloc de vanne (60) qui, conjointement avec les canaux (41, 42), établissent une communication fluidique entre la chambre de flexion (21) et la chambre d'extension (22),
tous les canaux écoulement (63, 64, 65, 66, 67) dans le bloc de vanne (60) sont couplés fluidiquement à un perçage (68),
un volume de compensation (70) est disposé dans le bloc de vanne (60), qui est couplé fluidiquement au perçage (68) par l'intermédiaire de l'un des canaux d'écoulement (67),
une vis (80) pourvue de canaux de communication (83, 84, 85, 87) est insérée dans le perçage (68), lesquels relient les canaux d'écoulement (63, 64, 65, 66, 67) entre eux,
**caractérisé en ce que**
la vis (80) fixe le bloc de vanne (60) au boîtier (10).

2. Actionneur hydraulique selon la revendication 1,
**caractérisé en ce que** le bloc de vanne (60) est fixé au boîtier (10) de manière amovible.

3. Actionneur hydraulique selon la revendication 1 ou 2,
**caractérisé en ce qu'**au moins deux vannes de réglage (51, 52) et au moins deux clapets anti-retour (53, 54) sont disposés dans le bloc de vanne (60).

4. Actionneur hydraulique selon l'une des revendications précédentes, **caractérisé en ce qu'**un raccord respectif (61, 62) pour un canal (41, 42) de la chambre de flexion (21) et de la chambre d'extension (22) est réalisé dans le bloc de vanne (60), et le raccord (61, 62) se subdivise en deux canaux d'écoulement (63, 64 ; 65, 66), dont l'un est pourvu d'une vanne d'étranglement réglable (51, 52) et l'autre d'un clapet anti-retour (53, 54).

5. Actionneur hydraulique selon l'une des revendications précédentes, **caractérisé en ce qu'**une vanne d'étranglement (51, 52) est placée en amont du point de couplage des canaux d'écoulement (63, 64, 65, 66, 67) dans le sens de l'afflux.

6. Actionneur hydraulique selon l'une des revendications précédentes, **caractérisé en ce que** précisément deux canaux (41, 42) sortent du boîtier (10).

7. Actionneur hydraulique selon l'une des revendications précédentes, **caractérisé en ce que** le bloc de vanne (60) est disposé sur une face frontale (11, 12) du boîtier (10).

8. Actionneur hydraulique selon l'une des revendications précédentes, **caractérisé en ce que** l'actionneur hydraulique est réalisé sous forme d'actionneur hydraulique linéaire comprenant une chambre cylindrique (20) et un piston (30), monté dans celle-ci de manière à pouvoir se déplacer longitudinalement, et une tige de piston (35) sortant du boîtier (10).
